# EUROPEAN PATENT APPLICATION

(11) **EP 4 635 527 A2**
(43) Date of publication of application: **22.10.2025**
(21) Application number: 25199859.7
(22) Date of filing: 08.12.2021
(51) Int. Cl.: A61L 27/38

(54) **THYMIC CONSTRUCTS AND USES THEREOF**

(30) Priority: 10.12.2020 GB 202019503
(62) Divisional of application: 21830725.4
(71) Applicant: The Francis Crick Institute Limited, London, Greater London NW1 1AT (GB)
(72) Inventor: Bonfanti, Paola, London, NW1 1AT (GB)
(74) Representative: Wilson Gunn

(57) **Abstract**

The present invention provides a method of producing a thymic construct suitable for implantation into a subject, the method comprising the steps of:
(i) providing an acellular scaffold;
(ii) seeding the acellular scaffold with thymic epithelial cells having mesenchymal properties; and
(iii) culturing the seeded scaffold to produce said construct.

The thymic epithelial cells are CD49f+ and at least one of the group consisting of VIM+, TE-7+ and CD90+. The invention further provides pharmaceutical compositions, uses and therapies using thymic epithelial cells having mesenchymal properties.

## Description

### Technical Field of the Invention

The present invention relates to thymic constructs, methods of manufacturing thymic constructs and uses thereof.

### Background to the Invention

The thymus is a primary lymphoid organ where haematopoietic progenitors are instructed to become functional T cells. It undergoes postnatal involution but remains in place throughout life.

The epithelial component of the thymus stroma derives from the endoderm, whereas mesenchymal stroma derives from the neural crest and in part from incoming vessels. Reflecting their fundamental role in immune regulation, many studies have provided insight into the origins and phenotypic complexity of thymic epithelial cells (hereinafter "TEC") and the molecules that mediate their activities, including AIRE and MHC Class II (MHCII) that are required for selecting T cells of diverse, non-auto aggressive specificities.

A core, long-established discriminator of TEC is their assignment as either cortical (cTEC) or medullary (mTEC), reflecting the anatomical regions to which the differentiated TEC contribute. However, while defined TEC and other stromal cell types have been studied in considerable detail, there is only incomplete molecular and cellular detail about how TEC and other cells collectively contribute to the complex three-dimensional (3D) organisation of the human thymus.

Since the thymus controls the development of both immune competence and tolerance, its functional dissection and subsequent reconstruction with desired cell populations might provide powerful tools applicable to many medical conditions, including primary or acquired immune deficiencies. Indeed, such is the clinical unmet need that cultivated thymus slices are currently used for transplantation into athymic patients, with many adverse complications.

Moreover, thymic therapeutic strategies might likewise be applicable to organ transplantation in which tolerance is a key obstacle to long-term graft acceptance.

Attempts to rebuild a fully functional thymus have so far met with limited success, possibly because of the thymus's inherent complexity. Moreover, there remains great uncertainty over the nature of postnatal epithelial stem/progenitor and mesenchymal cells including the capacity for extensive expansion *ex vivo.*

It would therefore be advantageous to provide the means to build a functional thymus using thymic tissue scaffolds.

It would also be advantageous to provide methods of producing thymic tissue constructs from decellularized thymus.

Furthermore, there is a need to be able to produce thymic tissue devices with phenocopied thymic cells, in order that the resultant constructs are as closely matched, phenotypically, to native *in vivo* thymus, as possible.

It would furthermore be advantageous to provide a single thymic cell type which enables repopulation of an acellular scaffold, especially which can be used without need to for addition of HSCs.

It is therefore an aim of embodiments of the present invention to overcome or mitigate at least one problem of the prior art, whether disclosed herein or not.

### Summary of the Invention

According to a first aspect of the invention there is provided a method of producing a thymic construct suitable for implantation into a subject, the method comprising the steps of:
(i) providing an acellular scaffold;
(ii) seeding the acellular scaffold with thymic epithelial cells; and
(iii) culturing the seeded scaffold to produce said construct,
wherein the thymic epithelial cells are CD49f+ and at least one of the group consisting of CD90+, VIM+ and TE-7+.

The acellular scaffold may be a decellularized tissue scaffold or a synthetic scaffold. Such scaffolds and methods for their production are well known in the art. For example, WO0214480 describes a number of scaffold categories: (1) non-degradable synthetic polymers; (2) degradable synthetic polymers; (3) non-human collagen gels, which are non-porous; (4) non-human collagen meshes, which are processed to a desired porosity; and (5) decellularized tissue.

An acellular scaffold typically does not comprise cells or cellular components. However, it will be appreciated that for example where a scaffold is used from a biological source, e.g. a decellularized scaffold, it is possible that some cells may remain on the scaffold e.g. after decellularization, as discussed below.

In one embodiment herein the scaffold is an artificial scaffold., which may be a synthetic or natural polymer scaffold.

Other synthetic scaffolds may be proteinaceous in nature e.g. primarily consist of purified proteins such as collagen. Non-synthetic scaffolds may also be proteinaceous in nature, or primarily consist of a collagenous extracellular matrix (ECM) from tissue.

The scaffold may be a 3-D printed scaffold, which may comprise any of the aforesaid materials.

Preferably the scaffold will be a decellularized (biological) matrix.

In some embodiments the scaffold comprises an acellular thymus scaffold.

In preferred embodiments the acellular thymus scaffold is an acellular whole thymus scaffold, preferably a whole thymus which has been decellularized; and in preferred embodiments the resultant construct is reconstructed thymus. In other embodiments the acellular thymus scaffold is an acellular portion of a thymus, such as a lobe or gland, for example.

The inventors have surprisingly found that it is possible to use particular thymic epithelial cells (TECs) to generate a functional thymus when seeded and cultured on an acellular scaffold. Surprisingly, the TECs exhibit epithelial-mesenchymal hybrid cellular function, capable of long-term expansion, and able to reconstitute an anatomic phenocopy of the native thymus, when combined with decellularized extracellular matrix (ECM) of thymus or other suitable scaffolds. This anatomical human thymus reconstruction is functional, as judged by its capacity to support, mature T cell development *in vivo* after transplantation into humanised immunodeficient mice.

The resultant construct may be considered an artificial organ, in particular an artificial thymus.

The TEC suitable for seeding onto acellular scaffolds display mesenchymal signatures, properties and/or function. By "mesenchymal signatures" it is understood that the TEC comprises one or more expression markers present in mesenchymal cells, particularly thymic mesenchymal cells, and by "mesenchymal properties" and "mesenchymal functions" we mean that the TEC display mesenchymal behaviour as well as epithelial behaviour, and are able to differentiate into different thymic cell types.

It has surprisingly been found that such "hybrid" TEC populations, having both epithelial and mesenchymal functions, can be isolated and used to create thymic constructs, without requiring further cell seeding with other cell types.

In some embodiments the populations of TEC showing mesenchymal properties are isolated, purified and expanded before step (ii). Isolation, purification and expansion may be performed by any suitable method as is customary in the art. The TEC are therefore preferably clonogenic TEC.

It has been found that using isolated and expanded cell populations provides more effective repopulation of the acellular scaffold, compared to known processes in which thymic cells are simply digested and applied to acellular scaffolds, or TECs are not isolated before culturing of the thymic cell population.

In some embodiments the TEC comprise medullary TEC (mTEC), cortical TEC (cTEC), or preferably a combination of mTEC and cTEC, especially a combination of mTEC and cTEC in which at least one of mTEC and cTEC, and preferably both, display mesenchymal expression markers

In other embodiments the TEC may be derived from precursor or stem cells, such as induced pluripotent stem cells which have been induced to differentiate into TECs, particularly mTEC and cTEC.

In preferred embodiments the TEC are substantially all CD49f+. In particularly preferred embodiments the CD49f+ TEC are also VIM+ and optionally TE-7+.

In some embodiments the TEC express CD90 (CD90+) and CD49f (CD49f+).

In preferred embodiments the TEC express CD49f (CD49f+) CD90 (CD90+) and VIM (VIM+).

In preferred embodiments the TEC are CD49f+ CD90+ VIM+ and TE-7+.

In some embodiments the TEC are substantially all CD90+. In some embodiments the CD90+ TEC also express VIM and optionally TE-7.

Unexpectedly, the inventors have found that the mesenchymal markers CD49f and CD90 (Thy1) are also expressed by a large proportion (>60%) of the mTEC and substantially all cTEC which show mesenchymal properties, and thus such selected TEC show hybrid epithelial-mesenchymal phenotype.

It has also surprisingly been found that isolated mTEC and/or cTEC expressing CD49f (CD49f+) and VIM (VIM+) and/or CD90 (CD90) have the ability to be subcultured and to be significantly expanded *ex vivo.* Thus, isolated (and preferably clonogenic) CD49f+, CD90+, CD90+ CD49f+, CD49f+ VIM+, and/or CD90+ VIM+ CD49f+ epithelial cells alone, can be pooled and used to subsequently seed and repopulate acellular thymus scaffolds, proliferating and differentiating to form an artificial thymus. It is particularly surprising that CD49f+, CD90+, VIM+ and TE-7+ cells can be seeded onto acellular scaffolds and differentiate to create a viable thymic construct, suitable for use in implantation into a subject, since many other TEC populations do not exhibit such properties or function.

In preferred embodiments step (ii) comprises seeding the acellular thymic scaffold with thymic epithelial cells and thymic interstitial cells (TIC). The thymic interstitial cells (TIC) may comprise one or more cell types selected from the group consisting of mesenchymal cells, fibroblasts and pericytes; and may comprise a combination of any two or all of the aforesaid cell types.

The acellular thymus scaffold may be co-seeded with TEC and TIC together.

The TEC and TIC may be seeded in a ratio of between 3:1 TEC:TIC to 10:1 TEC:TIC, preferably between 4:1 to 8:1 and most preferably around 5:1.

Whilst seeding of the acellular scaffold with TEC alone produces constructs in which TEC re-organise along the 3D extracellular matrix (ECM) structure from the subcapsular areas into the innermost regions of the scaffold, and the TEC are supported by the 3D (ECM) of the scaffold, reconstituted scaffolds with both thymic epithelial cells and thymic interstitial cells establish a more functional microenvironment supporting human T cell development from lymphoid progenitors *in vitro* and haematopoietic stem cells (HSC) *in vivo.* Thymic constructs produced by seeding TEC or TEC and TIC do not require any separate mesenchymal cell seeding or any other stem cell or precursor cell treatment.

In other embodiments the TEC may be seeded with non-thymic mesenchymal cells.

The TEC and TIC may be human TEC and TIC. Human TEC and TIC may be obtained from a live human donor prior to step (ii) or cadaver.

In embodiments wherein the acellular scaffold is an acellular thymus or part thereof, the thymus or part thereof may be human thymus or part thereof, obtained from a live human donor or cadaver.

Thus, by overcoming obstacles to constructing a functional thymus with only expanded, stromal cells, the present invention is believed to offer practical prospects for treating immune disorders including congenital athymia for which current therapies are limited.

The production method of the invention and thus the generation of a thymic construct is generally carried out *in vitro.* It will be appreciated however that further cell proliferation and/or differentiation and generation of the construct can occur after implantation *in vivo.* Thus, preferably the production of the construct is carried out *in vitro* until a construct is generated which is sufficiently populated with TEC that are sufficiently differentiated to allow successful implantation into a subject.

Further cellular proliferation in and/or on the scaffold can then subsequently occur e.g. after implantation. It will be appreciated therefore that a scaffold need not be entirely populated with the seeded cells to be useful for implantation into a subject. For example. it is possible that the scaffold has areas where seeded cells are not present, for example the scaffold may have seeded cells across at least 70, 80, 90, 95 or at least 99% of its surface.

The cells used in the present methods will typically be autologous i.e. originate from or are derived from the intended recipient of the tissue or organ construct generated by the method of the invention. However, cells for use in the method may also be allogeneic, i.e. obtained or derived from a subject who is not the recipient of the tissue or organ construct to be generated. Further, xenogeneic cells may be used, i.e. cells derived from a different species to the recipient of the tissue/organ construct. The cells may also be produced from pluripotent stem cells or induced pluripotent stem cells.

Preferably the seeding of the TEC (and TIC when present) is carried out by injection into the scaffold or by perfusion through the vasculature of the scaffold. Injection may be carried out in multiple stages. If a whole thymus scaffold is utilised, may be carried out by injection into part of the thymus scaffold (e.g. one or more lobes) or may be carried out by injection into multiple sites on the thymus scaffold. The cells may be delivered separately, simultaneously or sequentially. Sequential delivery may involve delivering the cell populations within at least 1, 2, 5, 10, 20, 30, 40, 50 or 60 minutes of each other, or within 1, 2, 3, 4, 5, 6, 7, 8, 9, 10, 11, 12, 14, 36 or 48 hours of each other.

The TEC or TEC and TIC may be injected into and/or onto each lobe of the acellular thymus scaffold at around 1-10 M, 1-8 M, 2-6 M or 2-3 M cells per 1 to 1000µl of medium per lobe.

The seeded scaffolds are typically maintained in vitro to allow for cell migration over the reticular ECM of the scaffold and initial differentiation, over a period of at least 1, 2, 3 or at least 4 days, and up to 21, 14, 12, 10, 9, 8 or 7 days, preferably around 4-7 days. Afterwards, donor cells such as stem cells and/or haematopoietic cells, may be delivered, preferably by injection, into or onto the constructs.

The acellular ECM scaffold or seeded ECM scaffold may additionally be treated to enhance adherence of cells to the scaffold and/or growth of cells on the scaffold. Such treatments may include the application of proteins such as growth factors or extracellular matrix proteins, for example, including one or more of collagen, elastin, fibronectin, laminin, or proteoglycans.

In some embodiments, step (ii) may comprise seeding of other thymic cells, in addition to the TEC and optional TIC cells. Other suitable thymic cells include thymic endothelial cells, fibroblasts, and hematopoietic stromal cells such as dendritic cells.

An "acellular" scaffold typically does not comprise cells or cellular components. However, it will be appreciated that for example where a scaffold is used from a biological source, e.g. a decellularized scaffold, it is possible that some cells may remain on the scaffold e.g. after decellularization, as discussed below.

In some embodiments the acellular scaffold comprises a decellularized scaffold, preferably decellularized thymic tissue, and more preferably decellularized whole thymus or a lobe thereof.

In one embodiment neonatal human donor thymus scaffold may be used. In another the scaffold may be derived from human cadaver. In yet another embodiment the scaffold may be derived from a living patient.

The scaffold may be xenogeneic i.e. it originates from or is derived from a donor of a different species than the recipient, for example, a human recipient.

In this connection, scaffolds suitable for decellularization include, *inter alia,* decellularized animal-derived scaffolds e.g. porcine-derived, rat derived or rabbit derived. For example, in a preferred embodiment the scaffold may be a decellularized rat, pig, sheep or rabbit thymus, more preferably pig.

Any suitable known decellularization method may be employed to provide the scaffold. In general, decellularization methods employ a variety of chemical, biochemical, and/or physical means to disrupt, degrade, and/or destroy cellular components and/or modify the matrix in which the cells are embedded so as to facilitate removal of the cells and cellular components, typically leaving an ECM scaffold. WO0214480 describes methods of decellularizing native tissues. The invention encompasses the use of decellularized scaffolds produced by any decellularization technique that removes substantially all of the cells while leaving the ECM substantially intact. Reference to leaving the ECM "substantially intact" refers to retaining the presence of at least 70, 80, 90, 95, 99 or substantially 100% of the matrix e.g. of the ECM.

In one embodiment decellularization of the thymus scaffold is performed by perfusing the tissue with at least one decellularization medium.

Suitable decellularization media include detergents, such as sodium dodecyl sulphate (SDS), sodium deoxycholate (SOC), detergents comprising hydrophilic polyoxyethylene-oxide and hydrophobic hydrocarbon moieties, such as Triton X-100 (RTM); enzymes, such as proteolytic enzymes, for example trypsin; and nucleases, for example deoxyribonucleases such as DNase I and ribonucleases such as RNase, and combinations thereof. Trisbutyl-n-phosphate (TBnP) may also be included in one or more decellularization media. TBnP is a solvent that disrupts protein-protein interactions.

The method preferably comprises perfusing the tissue with more than one decellularizing media. Suitably, the method comprises perfusing the tissue with at least one detergent and at least one nuclease. In some embodiments the method comprises separate steps of perfusing the tissue with a detergent and perfusing the tissue with a nuclease, and the detergent perfusion step may be performed before the nuclease perfusion step. After perfusion and decellularization the acellular thymus scaffold may be irradiated with gamma radiation; and may be stored at between 1-4°C until further use.

Each perfusion step may be performed at a temperature of between 15°C and 45°C, or between 20°C and 40°C. When the perfusion step comprises an enzyme perfusion step it may be performed at around 37°C, which is especially advantageous when the perfusion step includes a nuclease material. When the perfusion step is a detergent perfusion step it may be performed at a temperature of between 15°C and 40°C such as around ambient or room temperature.

In other embodiments, decellularization may comprise subjecting the thymic tissue or thymus to osmotic shock treatment.

According to a second aspect of the invention there is provided isolated thymic epithelial cells having mesenchymal properties for use in the regeneration of thymus, wherein the thymic epithelial cells are CD49f+ and at least one of the group consisting of CD90+, VIM+ and TE-7+.

The thymic epithelial cells may be for use in seeding onto a thymic scaffold for use in the construction of an artificial thymus or for use in repairing damaged thymus tissue.

In some embodiments there is provided thymic epithelial cells and thymic interstitial cells combined for use in the regeneration of thymus.

According to a third aspect of the invention there is provided isolated thymic epithelial cells having mesenchymal properties for use in the treatment of thymus, immune or autoimmune disorders, wherein the thymic epithelial cells are CD49f+ and at least one of the group consisting of CD90+, VIM+ and TE-7+.

According to a fourth aspect of the invention there is provided a pharmaceutical composition comprising isolated thymic epithelial cells having mesenchymal properties, and a pharmaceutically acceptable carrier, wherein the thymic epithelial cells are CD49f+ and at least one of the group consisting of CD90+, VIM+ and TE-7+.

The thymic epithelial cells of the second, third and fourth aspects of the invention may be as described herein above for the first aspect of the invention, and in particular may be TEC displaying mesenchymal, as well as epithelial, signatures, properties and/or function. In some embodiments the TEC express CD90 (CD90+) and CD49f (CD49f+). In preferred embodiments the CD49f+ TEC are also VIM+ and optionally TE-7+. In some embodiments the TEC express CD90 (CD90+) and CD49f (CD49f+). In preferred embodiments the TEC express CD49f (CD49f+) CD90 (CD90+) and VIM (VIM+). In preferred embodiments the TEC are CD49f+ CD90+ VIM+ and TE-7+. In some embodiments the TEC are substantially all CD90+. In some embodiments the CD90+ TEC also express VIM and optionally TE-7.

In some embodiments the TEC comprise medullary TEC (mTEC), cortical TEC (cTEC), or preferably a combination of mTEC and cTEC, especially a combination of mTEC and cTEC in which at least one of mTEC and cTEC, and preferably both, comprise mesenchymal expression markers.

In other embodiments the TEC may be derived from stem cells, such as induced pluripotent stem cells, which have been induced to differentiate into TECs, particularly mTEC and cTEC.

The TEC are preferably a mixture of isolated mTEC and isolated cTEC, preferably a mixture of isolated, purified and expanded mTEC and cTEC.

The pharmaceutical composition may further comprise isolated thymic interstitial cells (TIC).

The pharmaceutical composition may comprise TEC and TIC in a ratio of between 3:1 TEC:TIC to 10:1 TEC:TIC, preferably between 4:1 to 8:1 and most preferably around 5:1.

In some embodiments the TEC express CD90 (CD90+) and CD49f (CD49f+). In preferred embodiments the CD49f+ TEC are also VIM+ and optionally TE-7+. In some embodiments the TEC express CD90 (CD90+) and CD49f (CD49f+). In preferred embodiments the TEC express CD49f (CD49f+), CD90 (CD90+) and VIM (VIM+). In preferred embodiments the TEC are CD49f+, CD90+, VIM+ and TE-7+. In some embodiments the TEC are substantially all CD90+. In some embodiments the CD90+ TEC also express VIM and optionally TE-7. In some embodiments all of the TEC in the pharmaceutical composition express at least CD49f+, and substantially all of the TEC in the pharmaceutical composition may comprise CD49f+ and/or CD90+ thymic epithelial cells and a pharmaceutically acceptable carrier.

The pharmaceutical composition of the fourth aspect of the invention may be for use in the regeneration of thymus or for use in the treatment of thymus, immune or autoimmune disorders.

According to a fifth aspect of the invention there is provided a thymic construct obtained or obtainable by the method of the first aspect of the invention. The thymic construct may be an artificial thymus. The thymic construct may be for use in therapy; and may be for use in the implantation of a subject in need of a thymic construct implant, such as a subject with a damaged or missing thymus, for example.

Thus, in all aspects of the invention long-term expanding epithelial cells display a hitherto unprecedented hybrid epithelial-mesenchymal phenotype. These cells may repopulate whole organ scaffolds, phenocopying the unique 3D epithelial network of the thymus. Such reconstituted scaffolds have been found to establish a functional microenvironment supporting human T cell development from lymphoid progenitors in vitro and haematopoietic stem cells (HSC) in vivo.

The isolated cells, constructs and artificial organs may be for use in therapy and may be used in a method of surgery comprising implanting said construct, artificial organ or cells into a patient.

### Detailed Description of the Invention

In order that the invention may be more clearly understood embodiments thereof will now be described, by way of example only, with reference to the accompanying Figures, of which:

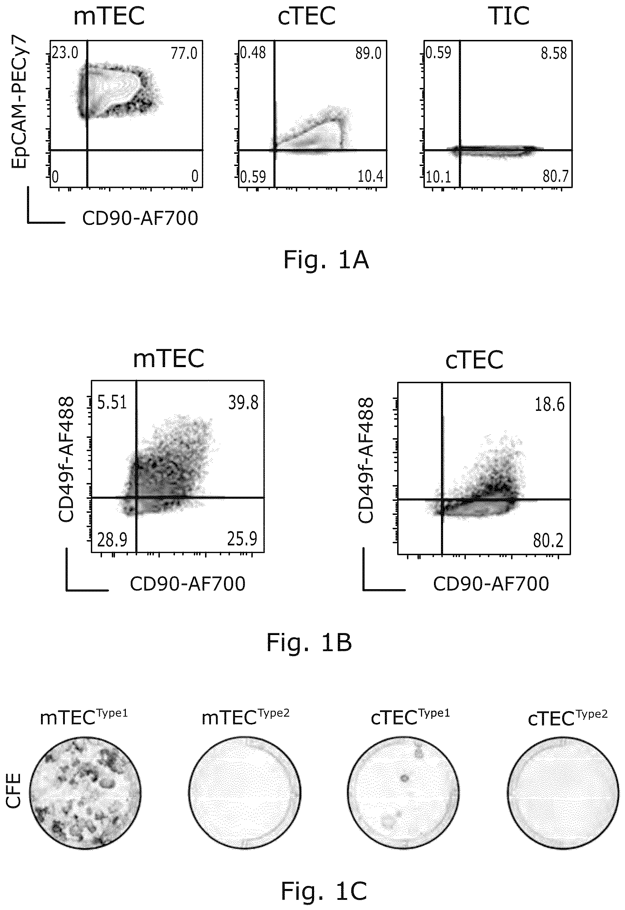

- Fig. 1A, 1B and 1C: illustrate the results of isolation of thymic epithelial (TEC) and mesenchymal cells (TMC). (**A**) FACS plots showing CD90 expression in enriched EpCAM^{high} mTEC, EpCAM^{low} cTEC and EpCAM⁻ TIC respectively. Gating was decided on negative unstained control value for each channel. Same thymus as in 1b. (**B**) Representative FACS analysis for CD49f and CD90 demonstrating CD49f⁺ (Type1) and CD49f⁻ (Type2) populations. Same thymus as in 1A. (**C**) Rhodamine-stained indicator dishes of sorted cTEC and mTEC subtypes (4000 events/dish) cultivated for 12 days, Colony forming Efficiency (CPE). mTEC^{Type1} is the most clonogenic population (2-4%) and cTEC^{Type1} (1-2%) is the clonogenic population of the cortex (n=4).

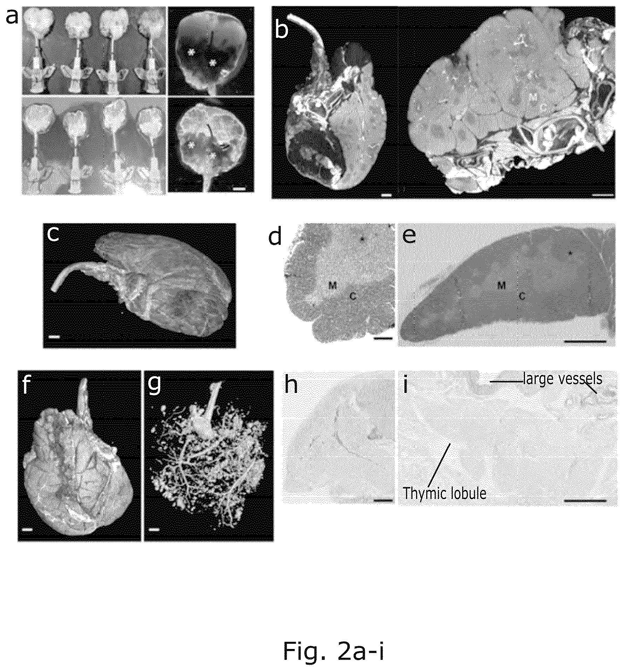

- Figure 2a to 2i: illustrate the results of whole organ thymus perfusion and decellularisation. (**a**) Gross appearance of cannulated rat thymi before (upper panels) and after (lower panels) decellularisation. A 24G cannula is inserted into the carotid artery and used to perfuse the organ with detergent and enzymatic solutions. Asterisks (*) indicate extra-thymic tissues that allowed connection between thymic tissue and cannula through the large blood vessels (n=120). Scale bar, 2 mm. (**b**) Micro-CT images of cannulated rat thymus showing extra-thymic tissues, large blood vessels and the 24G cannula entering the artery. Iodine contrast shows clear demarcation between cortex (C, bright) and medullary (M) areas; blood vessels (*) are represented by very bright areas between and inside the parenchyma (n=3). Scale bar, 1.5 mm. (**c**) Micro-CT 3D image of whole rat thymus cannulated where iodine contrast shows vasculature tree (segmented in red, n=2). Scale bar, 1.2 mm. (**d**) Masson's trichrome stain of a fresh rat thymus lobe staining in red keratins, in blue collagen and in pink cytoplasm. C, cortex; M, medulla (n=3 thymi). Scale bar, 250 µm. (**e**) Haematoxylin & Eosin (H&E) stain of a fresh rat thymus. C, cortex; M, medulla (n=3 thymi). Scale bar, 500 µm. (**f**) Micro-CT image of cannulated rat thymus showing the whole 2 lobes in 3D. Scale bar, 1.2 mm (**g**) Micro-CT image of a cannulated rat thymus injected with Microfil^{®} and thresholded to demonstrate perfusion of both thymic lobes. Scale bar, 1.2 mm (**h**) Masson's trichrome stain of a paraffin section of a decellularised rat thymus scaffold demonstrating collagen fibres (blue) and absence of keratins, muscle fibres and cell cytoplasm (n=3 scaffolds). Scale bar, 250 µm. (**i**) H&E of a decellularised thymus scaffold showing intact thymic lobule ECM and preservation of both large and small vasculature wall (n=4 scaffolds). Scale bar, 500 µm.

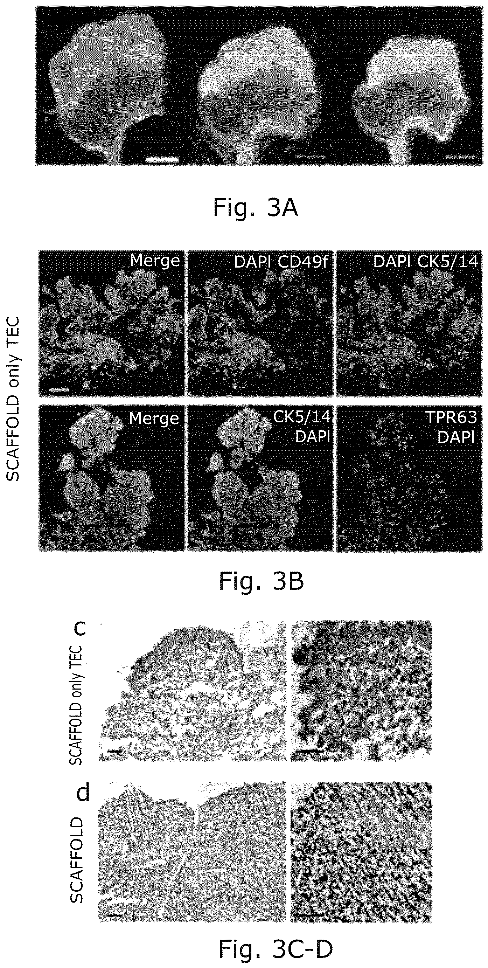

- Figure 3A to 3H: illustrate the results of the functional repopulation of whole organ thymus scaffolds. (**A**) Gross microscopy representative of a thymus scaffold before (left panel), soon after injection of stromal cells (middle panel) and following 4 days of culture (right panel). Thymic lobes from empty progressively increase density and get remodelled as shown by shrinking of the scaffold and increase tissue volume (n=60 repopulated scaffolds). Scale bar, 4mm. (**B**) Immunofluorescence labelling of thymic epithelial cells (TEC) grown within a decellularised scaffold demonstrating presence of CK5/14⁺, TPR63⁺ and CD49f⁺ TEC. Nuclei are stained with DAPI (n=4 repopulated scaffolds). Scale bar, 30 µm. (**C**) H&E staining shows histology of a scaffold repopulated only with TEC and cultivated for 5 days (n=4 repopulated scaffolds). Scale bar, 50 µm. (**D**) Haematoxyin & Eosin (H&E) staining of a scaffold repopulated with both expanded clonogenic TEC and thymic interstitial cells (TIC) and cultivated for 5 days prior to fixation and histological analysis. Stromal cells reorganise along the scaffold with a pattern similar to the one (**E**) observed in early (9- week post-conception, wpc) human foetal thymus (n=4 repopulated scaffolds and n=2 human foetal thymi). Scale bar, 100 µm. (**F**) Immunofluorescence labelling of TEC seeded together with TIC and grown within a decellularised scaffold demonstrating CK5/14⁺ cells localised in the sub-capsular region while CK8⁺ cells prevalently localised in the inner regions; TPR63⁺ TEC were mainly CD49f⁺. Nuclei are stained with DAPI (n=4 repopulated scaffolds). Scale bar, 100 µm. (**G**) H&E staining of a scaffold repopulated with TEC, TIC and haematopoietic progenitors after 7 days of culture (n=4 repopulated scaffolds). Scale bar, 100 µm. (**H**) Representative FACS analysis (n=6 repopulated scaffold in three independent experiments) of CD45-positive population isolated from repopulated scaffold seeded with triple negative

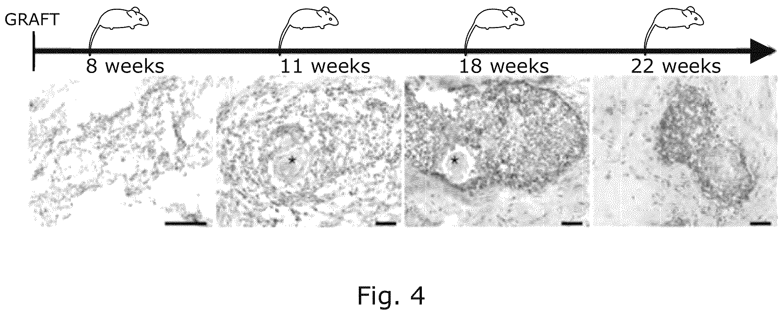

- Figure 4: (TN, CD3⁻CD4⁻CD8⁻) progenitors and co-cultured for 8 days. The FSC-A, SSC-A plot displays the presence of cells as well as of debris derived from the scaffold ECM during dissociation for release of haematopoietic cells (top left panel). Viable cells were about 90% of total cells (top mid panel). TN developed within the scaffold gave rise to double positive (DP) and single positive (SP) CD4 and CD8 expressing cells (top right panel, 5,000 cells). Live cells were positive for CD1a and negative for CD33 (bottom right panel). CD4 and CD8 were positive for CD3 and express TCRαβ (bottom mid and left panel). illustrates the results of repopulated thymus scaffolds maturing *in vivo* and promote functional T cell development, in the form of H&E staining of histological sections of thymic scaffolds harvested at different time points post-transplant in mice (8, 11, 18 and 22 wpt). (*) indicates Hassall's Bodies (HB); n=18 scaffolds in 3 independent experiments. Scale bar, 100 µm.

### Methods

### Animals

All animal procedures were in accordance with ethical approval and UK Home Office Project Licenses (PPL). (NSG) and NOD.Cg *Foxn1^{em1Dvs}.Prkdc^{scid}.Il2Rγc^{tm1Wjl}* (NSG-Nude, Stock No: 026263) were obtained from Jackson Laboratory. Mice were maintained on a 12 h light-dark cycle, ambient temperature 19/22°C, and humidity 45/65%. Sprague Dawley rats were kept in a breeding facility.

### Human tissues

Postnatal thymi were donated by patients (age range 3 days- 11 years old) undergoing cardiothoracic surgery at the Great Ormond Street Hospital, London, UK.

### Clinical thymus slices preparation and culture

After capsule removal, 8 to 15 grams of post-natal thymus tissue was processed with Stadie-Riggs microtome (Thomas Scientific), in order to obtain 1mm thick slices. Slices were individually mounted on nitrocellulose filters (Millipore), subsequently placed on Spongostan surgical sponges (Ferrosan Medical Devices) and bathed in culture medium (F12 (Gibco), 10% Heat-Inactivated Fetal Bovine Serum (Gibco) and 1% Pen-Strep (Sigma)) in 10-cm Petri dishes up to 21 days; culture media was replaced every day.

### CD34⁺ HSC purification and sorting

Mononuclear cells (MNCs) from two up to five CB collections were pooled and purified by Ficoll- Paque density centrifugation (GE Healthcare Life Sciences, Buckinghamshire, UK) followed by ammonium chloride red cell lysis. Density-separated CB MNCs were magnetically sorted for CD34 positivity via the EasySep Human CD34 Positive Selection kit (Stemcell Technologies) according to the manufacturer's instructions; alternatively, they were sorted by Fluorescent Activating Cell Sorting (FACS). Foetal Liver (FL) CD34⁺ were isolated from human foetal tissue ranging from 12 to 20 weeks post conception (wpc). Tissue digestion was performed at 37°C using an enzymatic solution (0.1 U/mL Collagenase A (Roche), 0.8 U/mL Dispase II (Roche) and 100 µg/ml DNase I (Roche) in RPMI, 2% FBS and 1% Penicillin/Streptomycin). Cells were pelleted and processed for ammonium chloride red cell lysis. FL MNCs were magnetically sorted for CD34 positivity.

### Thymocyte isolation and sorting

Human thymocytes were obtained by mechanical tissue dissociation of postnatal thymi. Enrichment of triple negative (TN, CD3⁻CD4⁻CD8⁻) cells was achieved by magnetic sorting using biotinylated anti-CD3, anti-CD4, anti-CD8a and anti-CD235ab antibodies (BioLegend) and Magnisort SAV negative beads (Invitrogen). The negative-selected fraction was processed for FACS purity sorting (CD11c⁻CD19⁻CD56⁻CD3⁻CD4⁻CD8⁻ cells; FACSAria III machine, BD Bioscience; FACSDiva 8.0.1 software).

### Isolation and cultivation of thymic stromal cell

Human thymi were obtained from 33 patients ranging in age from 3 days to 11 years who underwent open chest cardiac surgery. All thymi, independent of donor age, contained clonogenic TECs generating colonies that could be expanded extensively upon weekly passage. Thymic tissue fragments were dissociated to single cells by enzymatic treatment (0.4 mg/mL Collagenase D (Roche), 0.6 mg/mL Dispase II (Roche), 40 µg/mL DNAse I (Roche)) for about 30-45 minutes at 37°C. Cells were pelleted and re-suspended for cell counting. Freshly dissociated thymic cells were plated over a layer of lethally irradiated mouse fibroblast (3T3-J2). Alternatively, thymus single cell suspension was depleted of CD45 and CD235ab (red blood) expressing cells by immunomagnetic separation. The enriched fraction was stained and sorted using FACSAria III machine (FACSDiva 8.0.1 software) for cortical (EpCAM^{low}CD205⁺) and medullary (EpCAM^{high}CD205⁻) thymic epithelial cells (TEC) prior to cell culture over 3T3-J2 feeders. Epithelial cells expanded in cFAD medium composed by a mixture of 3:1 of DMEM-1X (Gibco) and F-12 Nut Mix (Gibco), supplemented with 10% Fetal Bovine Serum (Sigma), 1% penicillin and streptomycin (100X, Sigma), Hydrocortisone (0.4 µg/ml, Calbiochem), Cholera Toxin (10⁻¹⁰ M, Sigma), Triodothyronine (T3) (2x10⁻⁹ M Sigma) and Insulin (5 µg/ml, Sigma). Human epithelial growth factor (hEGF, 10 ng/ml, PeproTech) was added after three days of culture and then every other day at each feeding. TEC were plated with a density of 2000-4000 cells/cm², incubated at 37°C and 6% CO₂ reaching confluence between five and seven days of culture. TEC colony forming efficiency (CFE) assay was performed every other passage. Five-hundreds cells were obtained by a serial dilution and plated over lethally irradiated 3T3-J2 feeders in 60mm tissue culture dishes. After 12 days of culture, growing colonies were fixed in 4% Paraformaldehyde (PFA) and stained with Rhodamine-B (1%, SIGMA-ALDRICH) for 15 min. Colonies were scored under a dissecting microscope. Interstitial cells of the thymus (TIC) were derived either by sorting EpCAM⁻ stroma-enriched thymic population or by explants of human thymus. Fragments of thymic tissue were placed on 60mm dishes pre-coated with Matrigel^{™} (Corning), diluted 1:100 in Megacell medium (Sigma). Fragment were left to adhere for 30 min and then gently covered with Megacell medium supplemented with 2.5% FBS HI (Life Technologies), 1% Pen/Strept (Sigma), 1% L-glutamine (Life technologies), 1% Non-Essential Aminoacids (Life technologies), 100mM beta-Mercaptoethanol (Life Technologies) and basic-FGF (Sigma). After 7 day of culture, the cells outgrown from the explants were harvested, cultured at 37°C in a 6% CO₂ and 5% O2 atmosphere and passaged every 3-4 days, when TIC reached confluence.

### Lymphocyte culture and in vitro stimulation assay

Human CD45⁺/CD3⁺ sorted cells were cultured in RPMI 1640 (Gibco), Glutamax (Life Technologies) with 10% Foetal Bovine Serum (FBS, Life Technologies), 1% Penicillin-Streptomycin (Life Technologies) and supplemented with human recombinant IL-2 (50U/mL, R&D system) and IL-7 (5ng/mL, Invitrogen) up to 28 days in 96, 48 and 24 wells (Falcon). For expansion, anti-CD3/CD28 beads (Thermofisher) were added in the proportion of 1:1. Cytokine production was assessed by Phorbol Myristate Acetate (PMA) and *Ionomycin* (Io) stimulation assay: human CD45⁺/CD3⁺ sorted T cells and freshly isolated human thymocytes were incubated in RPMI, supplemented with 20% Human Serum Heat Inactivated (SIGMA-ALDRICH), Protein Transport Inhibitor Cocktail (500X, eBioscience), PMA (40 ng/mL, SIGMA-ALDRICH), and Io (4 µg/mL, SIGMA-ALDRICH) for 6h at 37°C. T cells were then washed with HBSS solution and stained for CD3, CD4 and CD8 (BioLegend), as well as APC-Cy7 fixable viability dye (Invitrogen), before fixation and permeabilisation with intracellular staining buffer kit (BioLegend) and intracellular staining with antibodies anti- IFNy, anti-TNFα and anti-IL-2 (BioLegend). Expanded and unstimulated T cells were used to set the FACS gates.

### Endothelial cells (HuVEC-VeraVec) culture

Human endothelial cells (Angiocrine, CAT: HVERA101) were cultivated on a layer of 0.1% gelatine (Merck) in Medium 199 (Gibco) supplemented with 20% Foetal Bovine Serum, FBS (Gibco), 1% Antibiotic/Antimicotic (ThermoFisher), 10mM HEPES (Gibco), 100 µg/ml Heparin (SIGMA-ALDRICH) and 50 µg/ml endothelial supplement ECGS (Millipore).

### Flow cytometry analysis

Single cell suspensions were stained with ad hoc antibody mix (Supplementary Table 1) in Hanks Balanced Salt Solution (HBSS, Life Technologies) supplemented with 2% FBS (Life Technologies) for 30 min on ice. DAPI (SIGMA-ALDRICH) or Zombie Live-Dead dye (Invitrogen) was used to discriminate live from dead cells. FACS phenotypic analysis was performed using Fortessa X-20 machine (BD FACSDiva8.0.1 software) and FlowJo^{™} software (BD Bioscience).

### RNA isolation and RT-qPCR

Cultured and freshly isolated cells were collected for gene expression analysis in either BL-Buffer from ReliaPrep^{™} kit (Promega) or in Trizol TRI Reagent (SIGMA-ALDRICH) following the manufactures instructions. Precipitated and dried RNA was re-suspended in nuclease free water (Qiagen). RNA concentration was measured using Nanodrop1000 (ThermoScientific) and RNA integrity (RIN) was evaluated using BioAnalyzer 2100 (Agilent). If needed, RNA was amplified using Ovation^{®} RNA Amplification System V2 (Nugen) following the manufacturer's instructions. Alternatively, RNA was converted into DNA with GoScript^{™} Reverse Transcriptase kit (Promega) according to the manufacturer's protocol. cDNA concentration was adjusted to 10ng/ul. Quantitative (q)PCR was performed using PCR master mix (PrecisionPLUS-R - Primerdesign Ltd) with low-ROX and Taqman qPCR probes (Integrated DNA echnology, Supplementary Table 2) in MicroAmp Fast Optical 96 well Reaction Plates (Applied Biosystems) using the QuantStudio 3 Real- Time PCR System (Applied Biosystems).

### Single-cell RNA sequencing - 10X genomics

The cell numbers were confirmed using an Eve automated cell counter (NanoEnTek). Where possible an appropriate volume for 10 000 cells was made up to 46.6ul with nuclease free water. For lower cell concentrations 46.6ul of cell suspension was loaded without further dilution. Reverse transcription and library construction were carried out following the Chromium single cell 3' reagent v3 protocol (10x Genomics) according to the manufacturer's recommendations. Total complementary-DNA synthesis was performed using 12 amplification cycles, with final cDNA yields ranging from approximately 3 ng/µl to 15 ng/µl. The 10x Genomics sequencing libraries were constructed as described and sequenced on an Illumina HiSeq 4000, with read lengths of 28-8-98.

### Bioinformatics data analysis of single-cell data

Raw sequencing data were processed using the CellRanger pipeline (10x Genomics). Count tables were loaded into R and further processed using the Seurat 3 R package⁵⁹. We removed all cells with fewer than 200 distinct genes observed or cells with more than 20% (samples mTEC1, mTEC2) or 30% (samples cTEC1, cTEC2) of unique molecular identifiers stemming from mitochondrial genes. Principal component analysis was then performed on significantly variable genes and the first 20 principal components were selected as input for clustering and UMAP based on manual inspection of a principal component variance plot ('PC elbow plot'). Clustering was performed using the default method from the Seurat package, with the resolution parameter set to 0.5. Area under the curve (AUC) summary intensity plots were prepared using the R-package AUCell.

### Bulk RNA sequencing library preparation and analysis

Total RNA was extracted from cultured cells using the RNeasy Micro Kit (Qiagen) and RNA total concentration was measured with a Nanodrop spectrophotometer. Total RNA was depleted of rRNA with Ribo-Zero, retrotranscribed using Superscript II reverse transcriptase (Invitrogen) and purified with RNA Clean Beads e e Ampure XP beads (Beckman Coulter). Libraries were prepared with the TruSeq Stranded Total RNA Sample Preparation Kit (Illumina) starting from 100 ng of RNA per sample and sequenced with the Illumina NovaSeq 6000 machine, paired ends, and a coverage of 35 million reads. RNA-Seq data were aligned with Salmon (GRCh38) and processed as follows. DEGs lists generation: analyses were performed with edgeR (v. 3.24.3). Significant DEGs were selected applying a 0.05 cutoff on FDR and a threshold of 1.5 on logFC. Genes comparison between different cell types: counts were normalized with the edgeR's function cpm() with the argument log=T. Then, data were visualized in a heatmap generated by the function heatmap.2() (gplots v.3.0.1.2).

### NanoString analysis

hCD45⁺CD3⁺ cells were sorted from the spleen of two NSG-nude mice at 10 and 18wpt (8081 and 1869 sorted events, respectively), lysed and hybridized to a chimeric antigen receptor-T cell panel for profiling 780 human genes at 65 °C overnight (NanoString Technologies). Hybridised samples were processed on an nCounter^{®} prep station and data were collected on an nCounter^{®} digital analyser (NanoString) following manufacturer's instructions. Raw data were imported into nSolver4.0 (NanoString) for data quality checks, background thresholding and normalisation. The 6 spiked-in RNA Positive Control and the 8 Negative controls present in the panel were used to confirm the quality of the run. Background level was determined by mean counts and 2× s.d. of negative control probes. Samples that contained fewer than 50% of probes above background or that had imaging or positive control linearity flags were excluded from further analysis. Probes that have raw counts below background in all samples were excluded from differential expression analysis to avoid false-positive results. Data were normalised by geometric mean of ten housekeeping genes present in the CAR-T Characterization panel.

### Histology

Human thymic tissues and scaffolds samples were fixed (for 2 hrs to overnight) in 4% PFA and processed for either cryo- or paraffin-embedding. For cryo-embedding, fixed tissue was equilibrated in sucrose 25% and embedded in O.C.T. compound (VWR). Cryosections (thickness, 7µm) were cut on a Leica Cryostat 3050. For paraffin-embedding, a Leica PelorisII tissue processor and Sakura Tissue-Tech embedding station were used. Paraffin section (thickness, 3-5 µm) were produced using ThermoFisher rotary microtome. Cryo- or Paraffin sections were stained with haematoxylin-eosin using an automatic station (Tissue-Tek Prisma), Masson's Trichrome Kit (Leica, Raymond A Lamb, BDH Chemicals), and Van Gieson Staining Kit (Millipore-Merck).

### Immunostaining

Tissue sections or coverslips were fixed in 4% PFA, blocked and permeabilised simultaneously using a solution of 5% Normal Donkey Serum (NDS, Jackson Immuno Research) in PBS, containing 0.5% of TritonX (TritonTMX-100, SIGMA-ALDRICH). Tissue sections or cells were incubated with primary antibodies 5% NDS, 0.01% TritonTMX solution overnight at 4 °C. Secondary antibodies were incubated at room temperature (RT) for 45 minutes. Nuclei were counterstained with Hoechst 33432 (10-6 M) or DAPI present in the Fluoroshield^{™} Mounting Medium (Abcam).

### Imaging

Phase contrast images of cultivated cells were acquired using an Olympus CK40 inverted microscope and Olympus SC50 camera. Live imaging of cultured cells was acquired by scope Zeiss Axiovert 135 with Hamamatsu Orca R2 camera (objectives 10x NA 0.25 Plan Neofluar). For histological images Zeiss Axioplan2 microscope with Zeiss Axiocam HRc color camera was used. Gross microscopy of organs was imaged using Zeiss StREO Discovery.V20 microscope and Zeiss Axiocam506 color camera. Zeiss LSM710 inverted confocal microscope and Zen Black software was used to acquire immunofluorescence images. Confocal images were processed using Fiji and Improvision Volocity LE software.

### Vascular microsurgery, perfusion and decellularisation

Male rats ranging from 150-220gr in weight were used as source of thymus organs. The thymus lacks a common artery to supply blood to the whole organ, therefore it is not possible to perfuse it by inserting a single cannula in the main blood vessel as for other organs³⁵. A microsurgical approach was used to obtain whole-organ thymus perfusion. Briefly, the vessels supplying blood to each lobe, were closed with a silk suture thread (size 6.0, F.S.T.) to seal the thymus in the following order: the right common carotid artery, the right subclavian artery, the right internal mammary artery, the right costocervical trunk, the right aortic arch, the left aortic arch, the left costocervical trunk, the left internal mammary artery. The left carotid artery was left open for subsequent cannulation and perfusion of the organ. Cannulated thymi were decellularised by perfusion of detergent-enzymatic treatment (DET). Organs were perfused with dH2O (18.2 mΩ/cm) for 96 hours at 4°C (0.2 ml/min) using a i150n peristaltic pump (*i Pumps*). DET was performed with 4% sodium deoxycholate (SDC; SIGMA-ALDRICH) at room temperature (RT) and 0.1mg/mL DNase-I (SIGMA-ALDRICH) in 2.5mM MgCl₂ (SIGMA-ALDRICH). Decellularised rat thymi (scaffolds) were gamma-irradiated with a dose of 1780 Gy and stored in PBS at 4°C for several weeks.

### Microfocus Compute Tomography (Micro-CT)

Rat thymi were extracted, cannulated and fixed in paraformaldehyde prior to immersion in potassium triiodide (I₂KI) contrast in a 1:1 ratio with the formalin solution, which was injected into the cannula, prior to immersion of the thymus into a falcon tube containing the same solution (approximately 20mls volume). I₂KI with a total iodine content of 63.25 mg/mL (iodine mass of 2.49 × 10⁻⁴ mol/mL) was used. Specimens were immersed in the contrast solution for 48 hours prior to scanning. Immediately prior to micro-CT examination, the specimens were rinsed in distilled water to remove excess surface iodine and padded dry using gauze. They were wrapped in Parafilm^{®}M and secured in a low-density plastic cylinder. Isotropic voxel sizes varied according to the geometric magnification achieved (inversely correlated with specimen size) and ranged between 5 and 9 µm. X-ray images were acquired using an XT H 320 microfocus-CT scanner with a multi-metal target (Nikon Metrology, Tring, UK). A tungsten target was used with an accelerating voltage of 100kV and current of 100 microamps. Following micro-CT examination (duration of approximately 90 minutes), the specimens were fixed in 10% formalin to prevent tissue degradation and aid with the removal of iodine prior to macroscopic examination. Images were reconstructed using CT Pro 3D (Nikon Metrology, Tring, UK) and post-processed using VG Studio MAX v 3.0 (Heidelberg, Germany).

### Scanning Electron Microscopy (SEM)

The samples were fixed in 4% formaldehyde / 2.5% glutaraldehyde (SIGMA-ALDRICH) in 0.1M Phosphate Buffer (PB) pH 7.4 for 5hours at RT. Samples were then washed in 0.1M PB, cut in approximately 5mm and cryoprotected in 25% sucrose, 10% glycerol in 0.05M PB overnight. Samples were then fast frozen and fractured in liquid nitrogen using a stainless-steel probe and then placed back into the cryoprotectant at RT to thaw. Samples were stained in 1% OsO₄ / 1.5% potassium ferricyanide, washed in H2O and dehydrated in a graded ethanol series, critical point dried with CO₂ using a Leica EM CPD300 and mounted on aluminium stubs using adhesive carbon tabs. The samples were mounted to present the fractured surfaces to the beam and coated with a thin layer of platinum using a Quorum Q150 R S sputter coater. Gross images of the samples were taken prior to SEM imaging on a Leica M205C stereomicroscope. SEM images were recorded with a FEI Quanta 250 PEG scanning electron microscope.

### DNA quantification

DNA from native rat thymi and decellularised organs was extracted with the PureLink Genomic DNA MiniKit (Invitrogen) according to the manufacturer's instructions. DNA samples were measured spectrophotometrically (NanoDrop^{™} 1000, ThermoFisher).

### Repopulation and in vitro culture of thymic scaffolds

Cell suspension in cFAD medium of cultivated TEC and TIC (ratio 5:1) were injected (2-3 M cells in100µl/lobe) using Insulin Syringes (Terumo, 29.5G) into decellularised rat thymi. Stromal cells used for repopulation of scaffolds were derived from 4 months and 6 months old donors. Seeded thymic scaffolds were cultured for five or six days in cFAD medium and then injected with TN (400,000) ± VeraVec (200,000) cells in 50µl volume of co-culture medium (DMEM 1X (Gibco), 10% FBS (SIGMA-ALDRICH), 1% penicillin and streptomycin (100X, SIGMA-ALDRICH), Triodothyronine (T3) (2x10⁻⁹ M SIGMA-ALDRICH), Insulin (5 µg/ml, SIGMA-ALDRICH) and Cytokines (Interleukin-7, 5ng/mL (Invitrogen), Stem Cell Factor 5ng/mL (Cell Signalling) and FLT3-L (5ng/mL, CellGS)). Scaffolds were reconstituted only with TEC (n=4), only TIC (n=2) or with TEC and TIC (n=5). Fully reconstituted scaffolds (with TEC, TIC and TN) were kept in co-culture medium (without cytokines, n=3) and cultured up to 14 days in cFAD medium.

### Bone marrow reconstitution and subcutaneous transplantation

NSG (8 to 12-week-old) mice were sub-lethally irradiated with 3.75 Gy from a ¹³⁷Caesium source (IBL 637 Gamma Irradiator). For primary engraftment, purified CD34⁺ cells (100K CB-CD34⁺ or 200K FL-CD34⁺/cells) were injected intravenously per mouse. Engraftment of human cells in the murine bone marrow was assessed at sacrifice. Sub-cutaneous implantation of the scaffold was performed in NSG mice 4 to 6 weeks post-CD34⁺ injection. Mice were anaesthetized with a 5-2% isoflurane oxygen gas combination for induction and maintenance. Buprenorphine 0.1 mg Kg⁻¹ was administered at the induction for analgesia. Under aseptic conditions, 1-3 midline incisions (0.4 cm) were performed on the back of the mice and scaffolds were inserted in lateral pockets⁴⁰. Mice were culled at different time points ranging from 1 week post-transplant (wpt) to 22 wpt.

Subcutaneous transplantation in three independent experiments was carried out on a total of 16 NSG mice, 14 of which had been humanized with CB-CD34 cells and 2 with FL-CD34 cells: all mice showed bone marrow reconstitution. The mice were implanted with 37 repopulated scaffolds, of which 34 were retrieved. Specifically, eight mice were implanted with repopulated scaffolds containing TIC, TEC, VeraVec and CD34⁺ cells (n=18); six mice received repopulated scaffolds without CD34⁺ cells (n=13), other two mice received scaffold without VeraVec cells (n=6). In addition, two mice received empty (n=2) and 2 mice TIC only scaffolds (n=6). No significant alterations in the extent of blood vessels correlated with the absence of endothelial cells in the scaffolds.

The NSG-nude female mice (8-12 week-old) were sub-lethally irradiated with 3.25 Gy and subsequently injected intravenously with purified human CD34⁺ cells. NSG-nude mice had a lower rate of humanisation than hairy littermates as established by bone marrow (BM) engraftment (4 out of 12 mice showed 27-55% hCD45⁺ cells against the 50-70% hCD45⁺ in 5 out 5 hairy littermates). Mice with no BM reconstitution were excluded from further analysis. BM and spleen were retrieved from humanised mice (one at 10wpt and one at 18wpt) engrafted with stroma- repopulated scaffolds and processed for flow cytometry analysis or sorting. Two humanised NSG-nude mice grafted with empty scaffolds showed no peripheral repopulation (no hCD3⁺ cells detectable in the spleen) at 18 wpt and 20 wpt.

### Scaffold FACS analysis and sorting

Repopulated natural scaffolds were mechanically and enzymatically dissociated using Collagenase I (2 mg/mL, SIGMA, C0130), Dispase II (1 U/mL, Roche) and DNAse I (80 □g/mL, Roche) in RPMI1640 and 2% FBS. At the end of digestion, cell suspension was passed through a cell strainer (100µm) and then stained with antibodies for FACS sorting or analysis. Sorting and/or analysis was performed on of a total of 16 Scaffolds of which 9 were repopulated with CD34⁺ HSC and 7 without CD34⁺ HSC. Four non-repopulated controls and two scaffolds with only TIC, CD34⁺ and VeraVec were harvested at 22wpt and 11wpt, respectively. Scaffolds were analysed and no thymocytes were detected. We found CD4/CD8 SP and DP in 14 repopulated scaffolds: in 7 out of 9 scaffolds repopulated with CD34⁺ HSC and in 7 out of 7 scaffolds repopulated without CD34⁺ HSC.

### Statistical analysis

Three or more biological replicates were performed for all experiments; the exact sample size (*n*) for each experimental group/condition, given as a discrete number. Statistical analysis was performed using two-way ANOVA non-parametric, unless stated otherwise. Plots and graphs were generated with GraphPad PRISM 8.

The above embodiment is/embodiments are described by way of example only. Many variations are possible without departing from the scope of the invention as defined in the appended claims.

### Examples

### Long-term in vitro expansion to identify clonogenic TEC

Human thymi were obtained from 33 patients ranging in age from 3 days to 11 years who underwent open-chest cardiac surgery. Thymus tissues were enzymatically dissociated to single cell suspension and plated over a lethally-irradiated feeder layer for the derivation of thymic epithelial cells (TEC). All thymi, independent of donor age, contained clonogenic TEC generating colonies that could be expanded extensively upon weekly passage. Subsequently, it was determined if the clonogenic TEC derived from the cortex and/or medulla. A cell-sorting strategy was used to dissect and prospectively isolate thymic stromal compartments including the epithelium (cortex and medulla TEC) and the interstitium (TIC). After a few cycles of enrichment, the CD45⁻ stromal population went from 0.02-2% (freshly dissociated tissue) to ~ 40-80%, allowing subsequent sorting on the basis of additional surface markers. EpCAM and CD205 expression levels were used to separate EpCAM^{high}CD205 medullary TEC (mTEC) from EpCAM^{low}CD205⁺ cortical TEC (cTEC). Unexpectedly, the mesenchymal marker CD90 (Thy1) was also expressed by a large proportion (>60%) of mTEC and approximately all cTEC, as well as by TIC (as shown in Figure 1A). CD49f, the *π*6-integrin which contributes to hemidesmosomes and is expressed by cells at the basal layer of stratified epithelia, was differentially expressed in both mTEC and cTEC populations (as shown in Figure 1B), providing a basis to sort for CD49f⁺ mTEC or cTEC ("Type1"), and CD49f⁻mTEC or cTEC ("Type2") sub-populations.

RT-qPCR was used to assess expression of established functional markers of freshly isolated medullary (e.g. *AIRE1*) and cortical (e.g. β-*5T*, *CD205*) epithelial cells, respectively. This resulted in differential expression in cortical *versus* medullary cells of cytokines and key transcription factors for thymus specification and/or thymopoiesis such as *FOXN1* and *PAX1* whereas Vimentin (*VIM*) was comparably expressed by all sorted populations examined.

To assess the colony-forming potential of freshly isolated Type1 and Type2 human cTEC and mTEC, they were plated over lethally-irradiated feeders, as described above. The Colony Forming Efficiency (CFE) assay consistently revealed that the highest clonogenicity resided in Type1 mTEC (CFE 2-4%) and Type1 cTEC (1-2%), while Type2 mTEC had a very low CFE (0.1-0.2%) and Type2 cTEC did not give rise to any expanding colonies (as shown in Figure 1C). Entirely consistent with their greater clonogenicity, Type1 mTEC and Type1 cTEC were the only epithelial subpopulations that could be expanded over serial passages. Type1 cTEC, although being less abundant than their medulla counterparts, robustly expanded in culture and could reach comparable numbers to Type1 mTEC. In order to define the transcriptional profile of thymic clonogenic cells, genome- wide RNA-sequencing of cultivated Type1 mTEC, Type1 cTEC was performed and also of TEC derived in culture without any sorting strategy. Expression of genes such as *SIX1, EYA1, HOXA3, PAX1, PAX9* detected by RNA sequencing of cultured TEC, confirmed that clonogenic TEC maintained thymic identity independently of their compartment of origin (cortical or medullary). Both cortical *(CSTV, KNICP3, CD274)* and medullary (*CHD1, EpCAM, CD24)* genes were retained by cultured cells. When analysed by RT-qPCR for transcription factors with documented importance for thymic epithelial cells, both mTEC and cTEC clonogenic cells expressed comparable levels of RNAs encoding TRP63, FOXN1 and signalling molecules such as SCF. mTEC and cTEC showed only 11 differentially expressed genes out of 14347 (0.08%) thus confirming that in culture they display a common phenotype. Thus, in subsequent experiments, we refer to "clonogenic TEC" whether or not they originate from medullary or cortical zones.

### Single-cell RNA sequencing defines cell clusters common to medullary and cortical populations in vivo

Freshly isolated subpopulations of mTEC and cTEC, Type1 and Type2, were subjected to single cell RNA sequencing (scRNA-seq), placing them into the broader context of other murine and human scRNAseq studies. This combined approach offered the opportunity for high resolution profiling of freshly isolated thymic epithelial cells enriched for clonogenic cells. When the mTEC and cTEC subpopulations were presented in a single UMAP, it was possible to annotate main cell subgroups among sorted epithelial cells, defining 15 cell clusters distinguishable on the basis of shared common features: cTEC grouped within three main clusters, while mTEC grouped within five, in addition to which we identified four TEC clusters common to both cortex and medulla ("comTEC"). Note that the remaining three clusters (residual cells) represented polymorphonuclear, dendritic cell, and thymocyte contaminants from the single-cell sorting. These main cell subgroups were validated by specific gene signatures of cTEC, mTEC or comTEC. The comTEC clusters included cells characterised by an Epithelial-Mesenchymal Transition (EMT) signature (cluster 12), proliferation markers (cluster 13), and poly-keratin expressing cells (cluster 14), while cluster 15 displayed a distinctive signature enriched in ion transporters. When the transcriptional signatures of comTEC and of specific mTEC and cTEC clusters were compared to the transcriptional profile of clonogenic TEC *in vitro,* it was evident that the latter expressed transcriptional signatures present in comTEC (proliferation, EMT and poly-keratins) but not in other clusters of freshly isolated mTEC or cTEC.

### TEC co-express epithelial and mesenchymal markers but are distinct from thymic interstitial cells

Based on the striking co-expression of CD90 (Thy-1) with EpCAM observed by FACS (as shown in Figure 1A) and also by the detection of an EMT signature in the comTEC cluster of freshly isolated TEC, this suggested the possibility that human thymic epithelial cells might be characterized by a distinctive epithelial-mesenchymal hybrid phenotype. Immunohistochemistry analysis was performed on postnatal thymi of our donors and clearly detected cells consistently co-expressing cytokeratins (CK) with mesenchymal markers such as CD90, vimentin (VIM) and to a less extent, the mesoderm marker TE-7, thus confirming at the protein level a hybrid phenotype expressed within healthy thymus tissue. In culture, clonogenic TEC were CD49f⁺, in active cell cycle (Ki67⁺) and double positive for CK5/14 and CK8/18. In contrast to all epithelia described in culture so far, but in line with what reported above, clonogenic TEC stably co-expressed mesenchymal markers such as CD90, VIM and TE-7. In addition, some TEC expressing TRP63, a transcription factor of the basal layer of stratified epithelia and a master regulator of thymic epithelial cells, were also positive for VIM *in vitro* and *in vivo.* Genome-wide transcriptomic analysis of cultured TEC confirmed the expression *in vitro* of the EMT signature defined by scRNA sequencing. Strikingly, expression of mesenchymal markers also correlated with high motility and migration that are typical of mesenchymal cells, as demonstrated by live-imaging of a TEC colony over 4 days in culture. Thus, the hybrid epithelial-mesenchymal phenotype of epithelial cells appeared to be a highly distinctive feature of the thymus *in vivo* and *ex vivo.* In addition to TEC, CD90⁺EpCAM⁻ interstitial cells were also expanded (as shown in Figure 1A). These cells did not grow under culture conditions designed for epithelial cells, but proliferated extensively for many passages in mesoderm culture conditions, thus allowing us to obtain billions of cells in only a few weeks. Immunofluorescence showed expression of mesenchymal markers such as TE7, VIM, PDGFR*β*, Chondroitin Sulfate Proteoglycan 4 (NG2) and Smooth Muscle Actin (αSMA) (Fig. 3d). FACS analysis revealed that these TIC consistently expressed PDGFRα, PDGFR*β*, CD90, CD146, and, to variable extents, NG2 and Alkaline Phosphatase (ALP), consistent with a perivascular phenotype. FACS analysis of expanding TIC showed no presence of either epithelial (CD49f⁺) or immune (hCD45⁺) cells. Finally, we compared the transcriptional profile of the cultured epithelial clonogenic cells and thymic interstitial cells from paediatric samples of different age by genome-wide RNA-sequencing. As expected, cultivated TEC appeared clearly distinct from the TIC as illustrated by the Principal Component Analysis (PCA1). Nevertheless, the data confirmed that the two cell types also share an exquisite similarity in the expression of genes related to EMT, including highly-expressed COL1A1, MMP2, TGFBR2 and FN1.

### Natural thymic scaffolds

Given the capacity of TEC and TIC to expand substantially under the culture conditions employed, it became possible to obtain billions of TEC and TIC within a relatively short time-period, establishing them as putative stem/progenitor cells with clinical applicability. In order to assess their capacity to functionally differentiate a unique approach was used to obtain thymic extra- cellular matrix (ECM) by whole-organ decellularisation that would provide a 3D structure for seeding thymic stroma and guide cellular re-organisation according to a physiological pattern.

Briefly, one carotid artery was left open for subsequent cannulation to allow perfusion of the organ, while all other arteries (downstream of the emergence of vessels reaching the thymus) were closed, as shown by gross microscopy and as detailed in the Methods section below (as shown in Fig. 2a, upper panels).

X-ray Microfocus-Computed Tomography (Micro-CT) of cannulated whole rat thymi was applied, to allow 3D visualisation of the 24G cannula sited in the carotid artery (as shown in Figure 2b). Micro-CT is a non- destructive technique that permits visualisation of the vasculature containing blood (white, dense areas) *via* iodine contrast; moreover, it facilitated clear demarcation of cortical (brighter areas, C) and medullary regions (M) through virtual segmentation, thereby demonstrating whole-organ 3D imaging of the thymus at high resolution (as shown in Figure 2b). When the parenchyma was rendered more transparent, the 3D whole organ micro-CT images allowed enhancement of the 3D thymic vascularisation segmented in red (as shown in Figure 2c). Standard histology by Masson Trichrome (MT) and Haematoxylin& Eosin (H&E) stains confirmed the distribution of cortical and medullary areas and vascular structures (as shown in Figure 2d and 2e).

This microsurgical approach allowed perfusion of both thymic lobes by injecting a Microfil^{®} compound through a single cannula, and, after solidification and fixation, we imaged the whole organ by Micro-CT (as shown in Figure 2f and 2g). Perfusion through the cannula of MilliQ-H₂0, followed by detergent and DNAse solutions allowed whole organ decellularisation over a 6-day period (as shown in Figure 2a lower panels). MT and H&E stains demonstrated complete acellularity of the organ, which displayed a fine, reticular, collagen-enriched ECM and preservation of the macro- and micro-vasculature (as shown in Figure 2h and 2i). Thymic ECM was characterised by colorimetric staining for ECM proteins that demonstrated preservation of elastin while confirming the absence of intermediate filaments, including keratins. Likewise, there was essentially no residual DNA after decellularisation. Scanning Electron Microscopy (SEM) showed distribution of ECM fibres at high resolution, and this was preserved in whole-organ scaffolds which were sterilised by γ-radiation and which could be stored in physiologic solution.

### Natural scaffolds facilitate functional thymic morphogenesis ex vivo

Scaffolds were injected with 4 to 6 million clonogenic TEC that had been expanded for four to six weeks. Repopulated scaffolds were then maintained in static conditions for up to 14 days in epithelial medium, over which time they showed scaffold remodelling and progressively increasing volumes (as shown in Figure 3A). TEC re-organised along the 3D ECM structure from the subcapsular areas (CD49f^{high}) into the innermost regions of the scaffold. Seeded TEC expressed CK5/14⁺ and/or CK8⁺; were in active cell cycle (Ki67⁺); were negative for the apoptotic marker, Caspase 3; and broadly expressed TRP63 (as shown in Figure 3B). These data demonstrate that TEC were supported by the 3D ECM.

Nevertheless, under these *in vitro* conditions and in the absence of any other cell type (as shown in Figure 3C), seeded cells resembled TEC detected in patients affected by primary immunodeficiencies wherein the distribution of thymic stroma is severely perturbed.

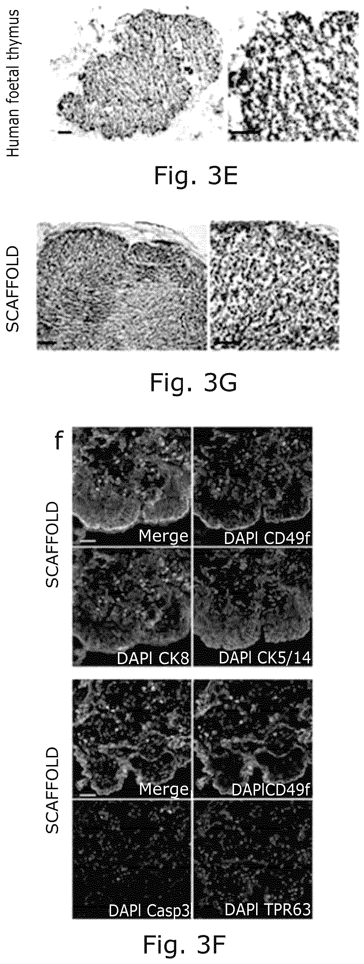

To address this issue, we next co-seeded expanded TEC together with cultured TIC. The TEC: TIC ratio was optimized at 5:1 in order to avoid interstitial cell overgrowth. Strikingly, after only 5 days of co-culture, the presence of TIC promoted a cordoned stromal organisation that largely phenocopied an early (9-week post-conception, wpc) foetal thymus (as shown in Figure 3D and 3E). TIC were found to be very important for reorganisation of TEC along the 3D ECM of the reconstituted scaffold. In the presence of TIC, cortical CK8⁺ TEC were mostly surrounded by a subcapsular-like layer of CK5/14⁺ TEC that expressed CD49f and TRP63, phenocopying the native thymus (as shown in Figure 3F). Scanning Electron Microscopy (SEM) of repopulated scaffolds confirmed cell-cell and cell-matrix interactions between TEC, TIC and decellularised ECM, as well as the preservation of vascular structures. The organised thymus stroma reconstituted *in vitro* from clonogenic cells was compared with clinical thymus slices that are cultured on a sponge up to 21 days, in order to remove donor thymocytes, before transplantation into athymic patients^{20,37}. The healthy status of the reconstituted stroma within our scaffolds seemed of particular relevance when considering that current clinical protocols for thymus slice preparation show disorganised stroma with variable survival after 14 and 21 days of culture, and equally importantly, residual CD3⁺ thymocytes that pose an overt risk of graft-versus-host disease for a transplant recipient.

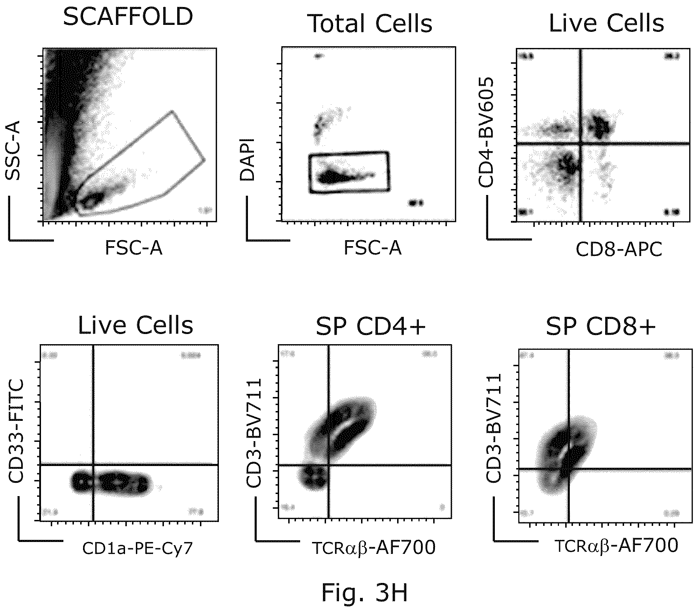

In order to assess the functional potential of the 3D thymic microenvironment, natural scaffolds were repopulated with TEC and TIC as described, and cultivated for 4-6 days before being injected with thymocyte precursors. Given the size (0.5-1 cm³) of the repopulated scaffolds, we cultured cells for a relatively short-term (up to 2 weeks) insufficient time *vis-a-vis* 5 to 6 weeks required for haematopoietic stem cell (HSC) lineage differentiation. To accommodate this, scaffolds were repopulated with flow cytometry-sorted Triple Negative thymocytes (TN, CD3⁻CD4⁻CD8⁻) characterized for the expression of early lymphoid markers CD1a, CD5, CD7 and CD31 (as shown in Figure 3G). Immune cells within repopulated scaffolds were negative for CD33, positive for CD1a and could differentiate toward both double (DP) and mature single positive (SP) CD4⁺ and CD8⁺ stages, resulting in a ratio of CD4⁺:CD8⁺ cells that phenocopies the ratio found in the native human thymus (as shown in Figure 3H). EpCAM⁺ TEC within the scaffolds expressed also HLA-DR which was not detected on TEC expanding in culture. In contrast, TN injected into scaffolds containing TIC-only stroma did not survive. In conclusion, it was possible to cultivate organ-size, 3D structures that supported the survival and appropriate spatial organisation of thymic stromal cells, that in turn facilitated thymocyte differentiation.

### Human thymus morphogenesis recapitulated in vivo

It was then determined whether repopulated thymus scaffolds might mature and sustain human T cell reconstitution from HSC *in vivo.* Scaffolds that had been repopulated and cultured *in vitro* for 5 to 6 days, were implanted subcutaneously into humanised NOD*.scid.*Il2Rγc*^{null}* (NSG) mice. NSG mice were sub-lethally irradiated and transplanted with highly purified CD34⁺ HSC from either human cord blood (CB CD34⁺) or foetal liver (FL CD34⁺) 4-6 weeks before they were implanted with thymus scaffolds co-seeded with stromal cells (TEC and TIC) and CD34⁺ HSC at defined ratios. The HSC, which can act as sources of human myeloid cells, were added to the grafts to accelerate thymus development, but were not essential.

Decellularised natural scaffolds previously seeded and cultivated *in vitro,* were grafted subcutaneously into NSG mice. At least 2 scaffolds were grafted into each mouse. Grafts were individually harvested at 1, 2, 8, 11, 18 and 22 weeks post-transplantation (wpt) (as shown in Fig. 4). Early time points were important to confirm stromal cell survival and re-organisation. At 8 wpt, stroma (TEC + TIC) seeded in natural scaffolds still showed a cordon-like distribution or compact epithelial structures by contrast to the unique 3D thymic epithelial reticulum (Fig. 4). However, by 11 wpt, ordered morphogenesis had progressed further, and more mature thymic structures were consistently detected among scaffolds seeded with stroma and CD34⁺ HSC. Among those were structures very similar to Hassall's Bodies (HB), an anatomic trait of human thymus that is much less evident in mouse (Fig. 4 HB indicated by asterisk,*).

Immunohistochemistry (IHC) for the epithelial marker E-Cadherin (ECad, red) and for the T cell receptor (TCR) (anti-CD3; green) demonstrated thymic morphogenesis with epithelial cells forming HB and interacting with CD3⁺ cells from the scaffold. Epithelial (CK5⁺) and mesenchymal (Vimentin⁺CK5⁻) cells, and angiogenesis (as detected by Endomucin immune-staining) were detected in scaffold transplants, lower panel). In some experiments VeraVec endothelial cells were seeded in addition to cultivated TEC and TIC since they have been reported to favour faster revascularisation in reconstituted BM niches. However, thymus morphogenesis and T cell development occurred independently of the presence of human endothelial cells at seeding, probably owing to murine vascularisation of the scaffold.

To determine whether the reconstituted scaffolds attracted circulating haematopoietic progenitors, morphogenesis was examined at 18-22 weeks post-transplantation (wpt), and found that thymic maturation was occurring in most repopulated scaffolds (Fig. 4) including those that were not injected with HSC prior to grafting. Moreover, AIRE expression progressively increased from 11 wpt to 22 wpt, and within several areas, with or without HB, there were scattered CD11c⁺ dendritic cells (DC), with HLA-DR expression detected by IHC on DC and by IHC and FACS on TEC.

In order to functionally characterise the implanted scaffolds, grafts were harvested, dissociated into single cells and analysed by flow cytometry. CD3⁺ cells were the prevalent phenotype within the hCD45⁺ population within the re-engineered thymic implants. The high percentage (30%-80%) of hCD45⁺ cells within each scaffold that were CD3⁺ indicates that the haematopoietic progenitors were subjected to an instructive thymic microenvironment provided by cultivated human stromal cells, since bone marrow (BM) of the same mice were by contrast primarily repopulated with CD33⁺ myeloid and CD19⁺ B lineage cells.

Flow cytometry showed that scaffolds were able to support thymocyte development (77.5% of all implanted scaffolds), with SP CD4⁺ and CD8⁺ cells expressing TCRαβ. Of note, the CD4:CD8 ratio for cells maturing in scaffolds favoured CD4⁺ SP cells (about 3:1), a feature of human thymocyte development that is not seen when circulating human BM-derived progenitors undergo differentiation within the endogenous thymi of NSG mice. Immature SP (ISP) and DP thymocytes were also present in different proportions within the scaffolds, demonstrating ongoing thymopoiesis, with consistent representation of DP cells. Nonetheless, their reduced presence by comparison to those reported in human thymi examined directly *ex vivo* may reasonably reflect the fact that further optimization will be required to establish the precise schedules and kinetics of differentiation in the native organ. In contrast, no CD3⁺ cells were retrieved from scaffolds repopulated with TIC, VeraVec, HSC CD34⁺ (without TEC) at 11wpt or empty scaffolds at 22wpt.

hCD45⁺CD3⁺ cells were sorted from thymus scaffolds and from the endogenous thymi of NSG mice at 18 wpt, and then interrogated the cells' functional potential *in vitro.* CD3/CD28 Dynabeads^{®} were used to induce the proliferation of sorted cells for from 12 to 28 days until sufficient cells were obtained for functional studies. Interestingly, the respective CD4/CD8 ratios of scaffold- associated or endogenous thymus-associated cells were sustained upon expansion in culture, indicating that this property had been differentially "instructed" by human and mouse thymic stroma. When stimulated *in vitro* with phorbol myristate acetate (PMA) and ionomycin (Io), the expanded CD4⁺ and CD8⁺ cells could produce IFNγ and TNFα, whereas IL2 was produced to a much lesser extent from cells developing in the human scaffolds than by T cells derived from the NSG thymi, (Fig. 6h). Of note, thymocytes freshly isolated from human thymus also respond to PMA- Ionomycin with low IL2 production.

Finally, the capacity of repopulated scaffolds to support peripheral T cell reconstitution was determined in an athymic NSG-*Foxn1^{null}* (NSG-nude) mouse, where the scaffolds were the only thymic stroma. NSG-nude mice were humanised 4 to 8 weeks prior to grafting. Littermate NSG (Hairy) mice were utilised as positive control for bone marrow (BM) and peripheral repopulation (spleen). Upon BM reconstitution no T cells (hCD3⁺) were detected in the periphery of these mice when transplanted with empty scaffolds. On the contrary, when NSG-nude mice were grafted with scaffolds previously repopulated *in vitro* with human thymic stroma, hCD3⁺ cells were detected in the periphery. Of note, hCD45⁺CD3⁺ cells from the spleens of NSG-nude mice were purified at two time points (10wpt and 18wpt) and analysed for expression of specific genes supporting TCR-activation including *TRBC1*/*2, TRAV19, NT5E, CD45RA, TRAC, CD69, PTPN6 (SHP-1), PTPRC, PPIA, CD22, LTB.*

### Discussion

The results above demonstrate that postnatal human thymus harbours epithelial (TEC) and interstitial cells (TIC) that can expand to clinically relevant numbers *in vitro,* suitable for contributing to the reconstruction of a human functional thymus *in vivo.* This demonstrates that a long-lived phenocopy of a human thymus utilising only postnatal, cultivated cells is possible.

The results show that both mTEC and cTEC expressing CD49f are enriched for clonogenic TEC with the ability to be subcultured and to be significantly expanded *ex vivo.* Interestingly, the expanding mTEC and cTEC mostly lost the signatures of their respective compartments of origin, seemingly consistent with there being a common progenitor present throughout thymic tissue. Unexpectedly, clonogenic TEC co-expressed genes and also displayed some behaviour typical of mesenchymal cells, which hybrid phenotype is believed to be a unique, cell-intrinsic feature of thymus stroma that is stably maintained over many passages in culture.

A distinct, *bona fide* interstitial cell population (TIC) could be extensively expanded *in vitro* and shared several features with other mesenchymal stromal cells and pericytes. Those cells were important in guiding TEC during morphogenesis, both *ex vivo* and upon transplantation *in vivo* since morphogenesis was defective in their absence. Possibly their role extends beyond supporting TEC development and regeneration, to synergizing with TEC in directly regulating T cell development.

3D natural ECM were shown to be very important for thymus morphogenesis from cultivated cells. The whole-organ perfusion-decellularisation approach described above allows preservation of the fine 3D ECM meshwork that is important for both *ex vivo* morphogenesis and long- term reconstitution *in vivo.* Moreover, human cultivated TEC gave rise to Hassall's Bodies, thus demonstrating their capacity to maintain key species-specific characteristics not previously reported for thymic progenitor differentiation *in vivo.* Functional genes such as AIRE and HLA-DR are also downregulated in the thymus stroma of human SCID patients *in vivo,* indicating that their expression is dependent on a complex microenvironment which includes cross-talk between the stroma and thymocytes. Hence, the conspicuous re-expression of AIRE and HLA-DR that was observed attests to the degree of reconstitution achieved in the methods of the invention. Crucial to demonstrating the functional competence of TEC and TIC upon *in vitro* expansion was their capacity to attract circulating human HSC, support T cell development and repopulate the periphery of athymic, NSG-nude mice. Species-specific stroma in an organ such as the thymus is crucial to its own function; The results show that a physiologic human CD4⁺/CD8⁺ ratio consistently achieved that has not been observed with hybrid mouse-human *in vitro* systems of human T cell development. Importantly, human T cells that developed *in vivo* within the human scaffolds were functionally different in their response to stimulation from those which developed in the mouse thymus, demonstrating the instructive capacities of the human stroma.

In conclusion, *in vivo,* long-lasting thymus were produced where both the haematopoietic and stroma compartments were of human origin. Such a system opens the possibility of addressing very many immunological questions ranging from T cell development, positive and negative selection with specific MHC-I and II, unconventional T cells (e.g. γδ), to the establishment and maintenance of tolerance. Future applications may include thymus transplantation in primary immune deficiencies such as athymic DiGeorge syndrome and *Foxn1^{null}* (nude) babies; the control of tolerance in congenital conditions as Autoimmune PolyEndocrinopathy - Candidiasis-Ectodermal Dystrophy (APECED) patients, and in immunosuppression-free organ transplantations.

Certain definitions of the invention are set out in the following numbered clauses.
1. A method of producing a thymic construct suitable for implantation into a subject, the method comprising the steps of:
   (i) providing an acellular scaffold;
   (ii) seeding the acellular scaffold with thymic epithelial cells having mesenchymal properties; and
   (iii) culturing the seeded scaffold to produce said construct.
2. A method according to clause 1 wherein step (ii) comprises seeding the acellular scaffold with both the thymic epithelial cells (TEC) and thymic interstitial cells (TIC).
3. A method according to clause 1 or 2 wherein the thymic epithelial cells comprise medullary thymic epithelial cells (mTEC), cortical thymic epithelial cells (cTEC), or preferably a combination of mTEC and cTEC.
4. A method according to clause 3 wherein the TEC and TIC are seeded in a ratio of between 3:1 TEC:TIC to 10:1 TEC:TIC, preferably between 4:1 to 8:1 and most preferably around 5:1
5. A method according to any preceding clause wherein the thymic epithelial cells are CD49f+ and preferably also at least one of the group comprising VIM+, TE-7+ and CD90+.
6. A method according to clause 5, wherein the thymic epithelial cells are CD49f+ and CD90+.
7. A method according to any preceding clause wherein the acellular scaffold is produced by decellularizing whole thymus or a part or lobe thereof.
8. A method according to clause 7 wherein the thymus, part or lobe is decellularized by perfusion with a least one decellularization medium selected from the group of a detergent, a protease and a nuclease.
9. A method according to any preceding clause wherein step (iii) comprises injecting or perfusing the thymic epithelial cells into and/or onto the scaffold.
10. Thymic epithelial cells having mesenchymal properties for use in the regeneration of thymus.
11. Thymic epithelial cells having mesenchymal properties for use in the treatment of thymus, immune or autoimmune disorders.
12. A pharmaceutical composition comprising isolated thymic epithelial cells having mesenchymal properties, and a pharmaceutically acceptable carrier.
13. A pharmaceutical composition according to clause 12 further comprising isolated thymic interstitial cells.
14. A pharmaceutical composition according to clause 12 or 13 wherein the thymic epithelial cells comprise medullary thymic epithelial cells (mTEC), cortical thymic epithelial cells (cTEC), or preferably a combination of mTEC and cTEC.
15. A pharmaceutical composition according to any one of clauses 12 to 14 wherein the TEC and TIC are seeded in a ratio of between 3:1 TEC:TIC to 10:1 TEC:TIC, preferably between 4:1 to 8:1 and most preferably around 5:1
16. A pharmaceutical composition according to any one of clauses 12 to 15 wherein the thymic epithelial cells are CD49f+ and preferably also at least one of the group comprising VIM+, TE-7+ and CD90+.
17. A pharmaceutical composition according to clause 16, wherein the thymic epithelial cells are CD49f+ and CD90+.
18. A thymic construct obtained or obtainable by the method of any one of clauses 1 to 9.
19. A thymic construct according to clause 18 for use in therapy.
20. A method of organ transplantation, the method comprising surgically implanting a thymic construct of clause 19 into a patient.

## Claims

1. A method of producing a thymic construct suitable for implantation into a subject, the method comprising the steps of:
(i) providing an acellular scaffold;
(ii) seeding the acellular scaffold with thymic epithelial cells having mesenchymal properties; and
(iii) culturing the seeded scaffold to produce said construct,
wherein the thymic epithelial cells are CD49f+ and at least one of the group consisting of CD90+, VIM+ and TE-7+.

2. A method as claimed in claim 1 wherein step (ii) comprises seeding the acellular scaffold with both the thymic epithelial cells (TEC) and thymic interstitial cells (TIC).

3. A method as claimed in claim 2 wherein the TEC and TIC are seeded in a ratio of between 3:1 TEC:TIC to 10:1 TEC:TIC, preferably between 4:1 to 8:1 and most preferably around 5:1

4. A method as claimed in any preceding claim wherein the thymic epithelial cells comprise medullary thymic epithelial cells (mTEC), cortical thymic epithelial cells (cTEC), or preferably a combination of mTEC and cTEC.

5. A method as claimed in any preceding claim, wherein the thymic epithelial cells are CD49f+ and CD90+.

6. A method as claimed in claim 5, wherein the thymic epithelial cells are CD49f+, CD90+ and VIM+.

7. A method as claimed in claim 6, wherein the thymic epithelial cells are CD49f+, CD90+, VIM+ and TE-7+.

8. A method as claimed in any preceding claim wherein the acellular scaffold is produced by decellularizing whole thymus or a part or lobe thereof, wherein the thymus, part or lobe is optionally decellularized by perfusion with a least one decellularization medium selected from the group of a detergent, a protease and a nuclease.

9. A method as claimed in any preceding claim wherein step (iii) comprises injecting or perfusing the thymic epithelial cells into and/or onto the scaffold.

10. Thymic epithelial cells having mesenchymal properties for use in the regeneration of thymus, wherein the thymic epithelial cells are CD49f+ and at least one of the group consisting of CD90+, VIM+ and TE-7+.

11. Thymic epithelial cells having mesenchymal properties for use in the treatment of thymus, immune or autoimmune disorders, wherein the thymic epithelial cells are CD49f+ and at least one of the group consisting of CD90+, VIM+ and TE-7+.

12. A pharmaceutical composition comprising isolated thymic epithelial cells having mesenchymal properties, and a pharmaceutically acceptable carrier, wherein the thymic epithelial cells are CD49f+ and at least one of the group consisting of CD90+, VIM+ and TE-7+.

13. A pharmaceutical composition as claimed in claim 12, wherein the thymic epithelial cells are CD49f+ and CD90+.

14. A thymic construct obtained or obtainable by the method of any one of claims 1 to 9.

15. A thymic construct as claimed in claim 14 for use in therapy.
